# EUROPEAN PATENT APPLICATION

(11) **EP 3 588 082 A1**
(43) Date of publication of application: **01.01.2020**
(21) Application number: 18305837.9
(22) Date of filing: 29.06.2018
(51) Int. Cl.: G01N 33/02, A23K 50/40, A61B 5/00

(54) **DEVICE FOR TESTING SNIFFING OF AN ANIMAL AND METHODS USING THE DEVICE**

(71) Applicant: Spécialités Pet Food, 56250 Elven (FR); Texas Tech University System, Lubbock, Texas 79409-2007 (US)
(72) Inventor: HALL, Nathaniel, Lubbock, TX Texas 79423 (US); PERON, Franck, 56250 ELVEN (FR); CAMBOU, Stéphanie, 56000 VANNES (FR)
(74) Representative: Regimbeau

(57) **Abstract**

The invention concerns a device (1) for testing sniffing of an animal, the device (1) comprising a receptacle (2) having a front opening (21) for receiving air flow produced by the animal.

The invention is characterized in that the device (1) further comprises an air flow sensor, which is configured to measure air flow in an air exit port (22) of the receptacle (2),
an odor line (4), able to introduce a selected odorant air stream (41) into the receptacle (2),
a calculator unit (6) for determination of a sniffing response of the animal for the selected odorant air stream (41) based on the air flow measured by the air flow sensor (3),
at least one presence sensor (5), and which is configured to detect the presence of the animal,
the calculator unit (6) is configured to command sending of the selected odorant air stream (41) in the odor line (4) towards the receptacle (2) as a response to the fact that the presence sensor (5) has detected the presence of the animal.

## Description

The invention concerns a device for the testing of sniffing of an animal and methods for using the device.

A field of application of the invention is the testing of dogs.

The worldwide pet food market is continually growing as the pet population becomes larger and larger over time, especially in developed countries. Facing an increasing public demand for high quality pet foods, the pet food industry is looking for supplying foods which have a high degree of palatability. Olfaction and taste combine to produce flavor, which is critical in determining the consumption of a food. In particular, olfaction is considered to be an integral role in the sensory experience of eating and food choice for dogs.

Dogs' ability to distinguish between different flavored foods requires olfaction. It would be useful to rapidly identify whether animals can quickly and spontaneously discriminate between two odorant substances.

Moreover, identifying the volatile organic compounds that influence olfactory attractivity is a continuous challenge for the pet food industry. Such technology would permit foods, or palatability enhancers added to foods, to be engineered so that appealing volatile organic compounds remain in the final product. In this context, it would be useful to know the concentration of an odorant substance at which an animal shows an interest in an odorant, if interested at all.

The first subject matter of the invention is a device for testing sniffing of an animal, the device comprising:
a receptacle having a front opening for receiving air flow produced by the animal, characterized in that the device further comprises an air flow sensor, which is configured to measure air flow in an air exit port of the receptacle.

According to an embodiment of the invention, the device further comprises an odor line (4), able to introduce a selected odorant air stream into the receptacle (2).

According to an embodiment of the invention, the device further comprises a calculator unit for determination of a sniffing response of the animal for the selected odorant air stream based on the air flow measured by the air flow sensor.

According to an embodiment of the invention, the device further comprises at least one presence sensor, which is configured to detect the presence of the animal.

According to an embodiment of the invention, the calculator unit is configured to command sending of the selected odorant air stream in the odor line towards the receptacle as a response to the fact that the presence sensor has detected the presence of the animal.

The invention thus enables to study animal olfaction through sniffing behaviors in various periods of the life of the animal. In order to generally test an animal's (and in particular a dog's) olfaction, a new device is here proposed which enables the analysis of sniffing behaviors of animals in response to odorants delivered mainly ortho-nasally. Indeed, the inventors observed that sniffing parameters determined with this device are highly relevant to analyze olfactory behaviors.

According to an embodiment of the invention, the presence sensor comprises an infrared beam sensor to project an infrared beam situated in front of the receptacle or in the receptacle,
wherein the infrared beam sensor comprises an emitter to emit the infrared beam and a detector to detect the infrared beam,
wherein the presence sensor is configured to detect the presence of the animal when the emitter is in an operating state, in which the emitter emits the infrared beam directed to the detector and the infrared beam is not detected by the detector.

According to an embodiment of the invention, the presence sensor is embodied in the calculator unit and is configured to detect the presence of the animal when variations of the air flow in the air exit port correspond to inspiration and/or expiration of the animal.

According to an embodiment of the invention, the air flow sensor comprises an air flow pressure sensor to measure a pressure of the air flow in the air exit port,
wherein the presence sensor is at least partly embodied in the calculator unit and is configured to detect the presence of the animal when the pressure of the air flow in the air exit port has a pressure amplitude which is above a predetermined pressure amplitude threshold.

According to an embodiment of the invention, a first presence sensor and a second presence sensor are provided as presence sensors,
wherein the first presence sensor comprises an infrared beam sensor to project an infrared beam situated in front of the receptacle or in the receptacle, wherein the infrared beam sensor comprises an emitter to emit the infrared beam and a detector to detect the infrared beam, wherein the first presence sensor is configured to detect a first event when the emitter is in an operating state, in which the emitter emits the infrared beam directed to the detector and the infrared beam is not detected by the detector,
wherein the air flow sensor comprises an air flow pressure sensor to measure a pressure of the air flow in the air exit port,
wherein the second presence sensor is at least partly embodied in the calculator unit and is configured to detect the presence of the animal when the pressure of the air flow in the air exit port has a pressure amplitude which is above a predetermined pressure amplitude threshold after a predetermined duration after the first event.

According to an embodiment of the invention, the odor line is connected to an intermediate opening of the receptacle, situated between the front opening and the air exit port.

According to an embodiment of the invention, the air flow sensor is of pneumo-tachometer type and measures a differential air pressure value indicating inspiration and expiration through the air exit port,
the calculator unit or a computer connected to the calculator unit is configured to determine as sniffing response a mean instantaneous sniffing frequency being a frequency of peaks of the differential air pressure values measured by the air pressure sensor through the air exit port.

According to an embodiment of the invention, the calculator unit or a computer connected to the calculator unit is configured to determine as sniffing response:
- inspiration peaks and expiration peaks as peaks of the differential air pressure values in the air exit port,
- an instantaneous inspiration frequency of each inspiration peak by identifying the time Tᵢₙₛₚ elapsed between successive inspiration peaks and calculating the reciprocal of this time Tᵢₙₛₚ as being the instantaneous inspiration frequency,
- an instantaneous expiration frequency of each expiration peak by identifying the time Tₑₓₚ elapsed between successive expiration peaks and calculating the reciprocal of this time Tₑₓₚ as being the instantaneous expiration frequency,
- the mean instantaneous sniffing frequency or frequency of peaks as being the overall mean of the instantaneous inspiration frequencies and instantaneous expiration frequencies.

According to an embodiment of the invention, the air flow sensor is of pneumo-tachometer type and measures a differential air pressure value indicating inspiration and expiration through the air exit port,
the calculator unit or a computer connected to the calculator unit is configured to determine as sniffing response :
zero crossings of the differential air pressure value measured by the air pressure sensor,
peaks of the differential air pressure value measured by the air pressure sensor,
a volume of sniffing being an area between two successive zero crossings for each peak of the differential air pressure values measured by the air pressure sensor,
a mean volume of sniffing being a mean of the volume of sniffing of the peaks.

According to an embodiment of the invention, the device further comprises at least a container containing an odorant substance and an air sending unit to send an air stream through the container and to introduce the selected odorant air flow coming from the container into the odor line.

According to an embodiment of the invention, the device comprises at least two respective containers containing odorant substances, wherein the air sending unit comprises selectors enabling selection of which of the containers, called selected container, the air sending unit sends air to introduce the selected odorant air flow coming from the selected container into the odor line.

According to an embodiment of the invention, the device comprises an air flow rate regulator to enable the setting of different air flow rates in the odor line.

According to an embodiment of the invention, the calculator unit is configured to send the selected odorant air stream in the odor line to the receptacle after at least a first prescribed duration after the instant of detection of the presence of the animal by the presence sensor.

A second subject matter of the invention is a method to determine a minimal concentration threshold of an odorant at which an animal shows interest. In this method, at least a mean instantaneous sniffing frequency of a sniffing response of the animal and/or a mean volume of sniffing of a sniffing response of the animal is determined by the calculator unit of the device as described above for different concentrations of the odorant in the selected odorant air stream. The minimal concentration threshold of the odorant is determined by a concentration of odorant above which the mean instantaneous sniffing frequency and/or the mean volume of sniffing increases above a first determined value or decreases below a second determined value.

A third subject matter of the invention is a method to test discrimination of at least two different odorants by an animal. In this method at least a mean instantaneous sniffing frequency of a sniffing response of the animal and/or a mean volume of sniffing of a sniffing response of the animal is determined by the calculator unit of the device as described above for each of the respective different odorants in the selected odorant air stream,
wherein the respective mean instantaneous sniffing frequencies having been determined for the respective different odorants are compared one to another to indicate non-discrimination of the animal between the different odorants when the respective mean instantaneous sniffing frequencies are in a same determined range and to indicate discrimination of the animal between the different odorants when the respective mean instantaneous sniffing frequencies are not in the same determined range and/or
the respective mean volume of sniffing having been determined for the respective different odorants are compared one to another to indicate non-discrimination of the animal between the different odorants when the respective mean volume of sniffing are in the same determined range and to indicate discrimination of the animal between the different odorants when the respective mean volume of sniffing are not in the same determined range.

The invention will be more clearly understood from the following description, given solely by way of non-limiting example in reference to the appended drawings, in which:
- Figure 1 is a block diagram schematically illustrating a device according to an embodiment of the invention,
- Figure 2 is a perspective view schematically illustrating a receptacle of a device according to an embodiment of the invention,
- Figure 3 is a cross section view schematically illustrating a receptacle of a device according to an embodiment of the invention,
- Figure 4 is a perspective view schematically illustrating the device according to an embodiment of the invention,
- Figure 5 shows schematically an air sending unit of the device according to an embodiment of the invention,
- Figure 6 shows schematically a part of the air sending unit of the device according to an embodiment of the invention, which is connected to a container of the latter,
- Figures 7A and 7B are diagrams showing in ordinates the log proportion of baseline sniffing frequency determined by the device according to an embodiment of the invention as a function of odor dilution of different odorant substances in abscissas,
- Figures 8, 9 and 10 are diagrams showing in ordinates the sniffing frequency determined by the device according to an embodiment of the invention as a function of trials in abscissas for different odorants,

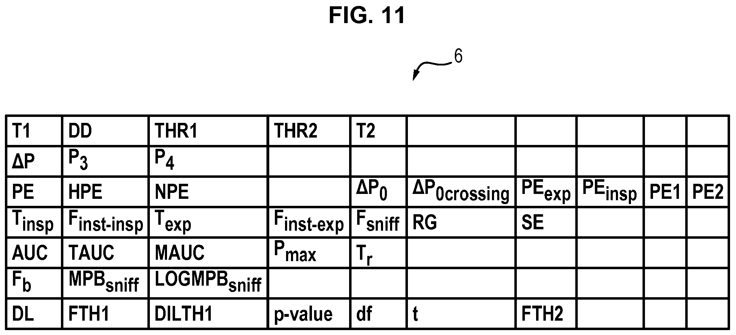

- Figure 11 shows parameters determined or calculated by a calculator unit 6 or used otherwise of the device according to embodiments of the invention.

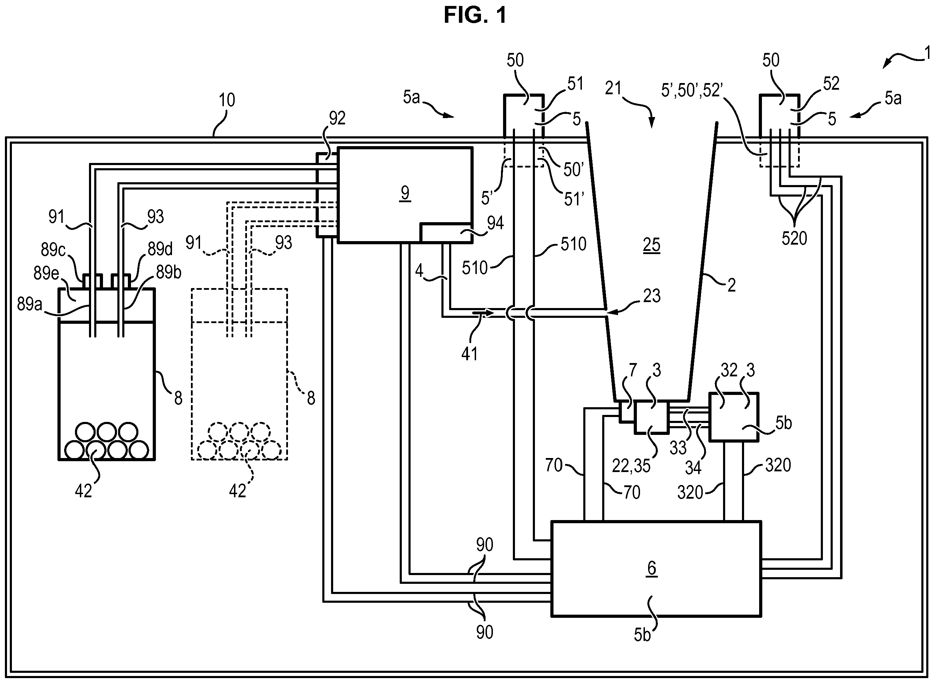

In figures 1 to 4, the device 1 for testing sniffing of an animal comprises a receptacle 2 having a front opening 21 for receiving air flow produced by the animal. The receptacle 2 has an inner wall 24 defining an inner cavity 25. The receptacle 2 may be called a mask in the examples below. The cavity 25 is of sufficient size so that the nose of the animal can be inserted in the receptacle 2 through the front opening 21. For example, the animal is a domestic animal or a pet, for example a domestic dog. The receptacle 2 is attached to a fixed casing 10, for example intended to be placed on the ground. The device 1, the receptacle 2 and the casing 10 are not intended to be carried by the animal but form a fixed or immobile unit. The front opening 21 is for example attached in a front wall 101 of the casing 10, and is opened to the outside and free to be accessed by the animal. The front opening 21 is turned for example with an inclined angle ANG comprised between 0° and 90° from bottom to top of the front wall 101 of the casing 10. The casing 10 encloses or carries the other parts of the device 1, which are described below.

In an embodiment, the casing 10 may be opened by moving or raising the front wall 101 or moving other walls of the casing 10. This enables to place said other parts of the device 1 in the casing 10.

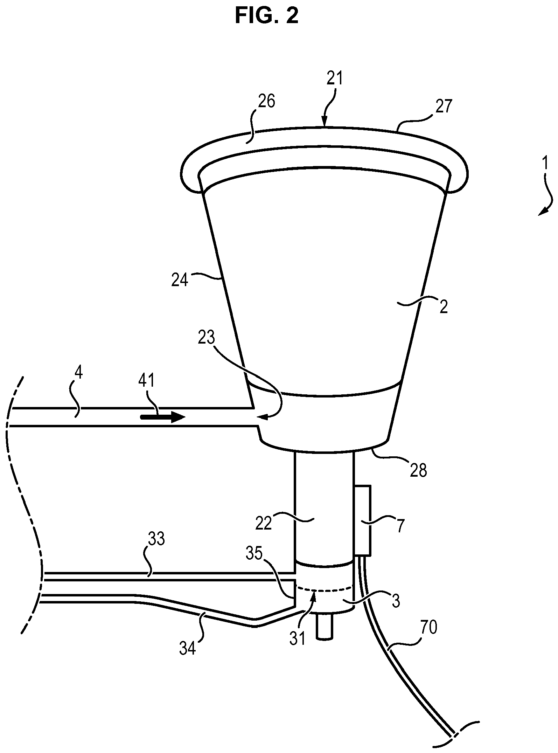

An air flow sensor 3 is provided to measure air flow in the receptacle 2, caused by the animal breathing and/or sniffing in the receptacle 2. The air flow sensor 3 is connected to an air exit port 22 of the receptacle 2, situated at a distance from the front opening 21 and thus measures air flow in the air exit port 22. For example, in figure 2, the front opening 21 is situated at the top end 27 of receptacle 2 and the air exit port 22 is situated at the bottom end 28 of receptacle 2 which is at a distance from the top end 27. The air flow sensor 3 may comprise an air flow pressure sensor 32 to measure a pressure of the air flow in the air exit port 22. This air flow pressure sensor 32 may be a differential air pressure sensor 32.

In an embodiment shown on figures 1 to 3, the receptacle 2 has a shape narrowing from the front opening 21 to the air exit port 22. For example, the wall 24 of the receptacle 2 can be of conical shape. For example, the front opening 21 can be delimited by an outer ring 26 attached to the wall 24. The outer ring 26 can be circular.

An odor line 4 is provided to introduce a selected odorant air stream 41 into the cavity 25 of the receptacle 2 when the odor line is activated. The odor line is able to send the odorant air stream 41 carrying a selected odor to the nose of the animal when the latter is inserted in the cavity 25. In an embodiment shown on figures 1 and 2, the odor line 4 is connected to an intermediate opening 23 of the receptacle 2, which is situated between the front opening 21 and the air exit port 22 and communicates with the inner cavity 25. This odor line 4 forms with the other means 2, 8, 9 described below an olfactometer.

The device 1 comprises a calculator unit 6 for treatment of the air flow measured by the air flow sensor 3 for assessment of a sniffing response of the animal for the selected odorant air stream 41. In an embodiment shown on figures 1 to 3, a presence sensor 5 configured to detect the presence of the animal is fixed relative to the receptacle 2 or casing 10.

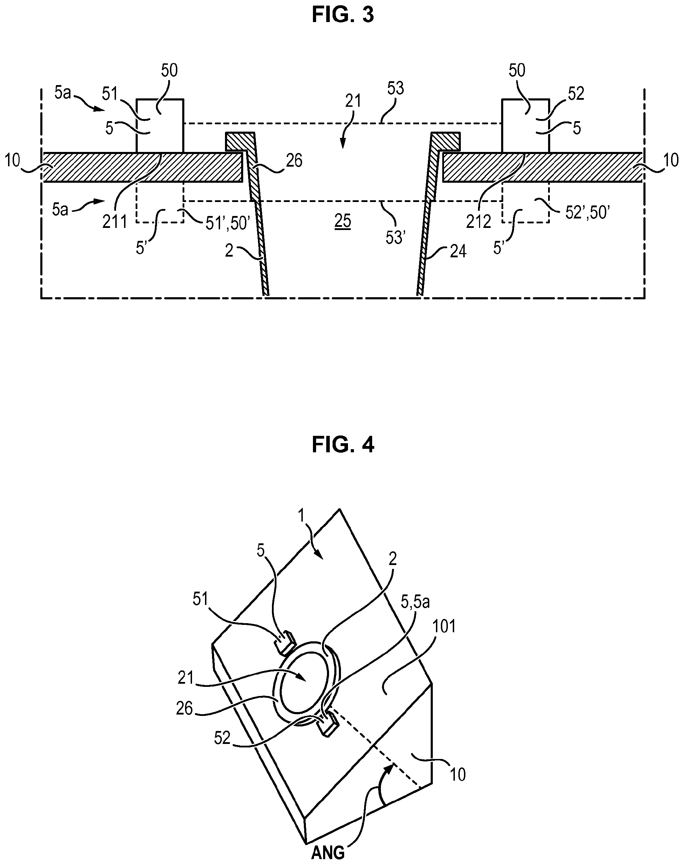

In an embodiment shown on figures 1, 3 and 4, the presence sensor 5 may be an infrared beam sensor 50 for example situated in front of the front opening 21 to detect the presence of the animal in front of the front opening 21. Of course, the infrared beam sensor 50 could be an infrared beam sensor 50' situated behind the front opening 21 and behind the wall 101 inside the casing 10, as shown by reference 50' in figures 1 and 3. The infrared beam sensor 50 and/or 50' is called first presence sensor 5, 5a. The presence sensor 5a is connected through wires 510, 520 to the calculator unit 6 to send its measurement to the latter.

In an embodiment shown on figures 1 to 3, the calculator unit 6 is configured to send the selected odorant air stream 41 in the odor line 4 towards the cavity 25 of the receptacle 2 as a response to the fact that the presence sensor 5 detects the presence of the animal, i.e. in the above example when the nose of the animal is detected at the front opening 21 or is inserted in the front opening 21. The device 1 then automatically sends the odorant air stream to the animal, each time the animal is detected by the presence detector 5, i.e. in the above example each time the animal inserts its nose in the front opening 21. The device 1 then enables an automatic measurement of air flow caused by the animal in the receptacle 2 by the means 3. The air flow measured by the air flow sensor 3 quantifies the sniffing of the animal to smell the odor presented in the receptacle through the odor line 4.

The device 1 enables to correlate the measurement of air flow caused by the animal in the receptacle 2 by the means 3 to the odor present in the odorant air stream introduced in the receptacle 2.

According to an embodiment, there is provided a method to determine a minimal concentration threshold of an odorant, above which an animal shows interest, in which at least a mean instantaneous sniffing frequency F_{sniff} of a sniffing response of the animal is determined by the calculator unit 6 of the device 1 for different concentrations of the odorant in the selected odorant air stream 41, wherein the minimal concentration threshold (DILTH1 in example 1) of the odorant is determined by a concentration of odorant above which the mean instantaneous sniffing frequency F_{sniff} increases above a first determined value FTH1 (determined according to experimental conditions) or decreases below a second determined value FTH2 (determined according to experimental conditions). According to another embodiment, there is provided a method to determine a minimal concentration threshold of an odorant, above which an animal shows interest, in which at least a mean volume of sniffing MAUC of a sniffing response of the animal is determined by the calculator unit 6 of the device 1 for different concentrations of the odorant in the selected odorant air stream 41, wherein the minimal concentration threshold DILTH1 of the odorant is determined by a concentration of odorant above which the mean volume of sniffing MAUC increases above a first determined value FTH1 (determined according to experimental conditions) or decreases below a second determined value FTH2 (determined according to experimental conditions).

The invention enables to determine a threshold of concentration of one particular odorant substance 42, above which the animal has (positive or negative) interest to the odor, as shown in example 1. In other words, the device according to the invention enables to measure an odor threshold concentration at which dogs notice and investigate the odorant. This may be measured by the volume of sniffing (see AUC below) and/or frequency of sniffing (see F_{sniff} below) of the animal measured by the air flow sensor 3. The threshold of concentration may be determined, by the fact that there is a sudden important variation or raise or decrease in frequency and/or volume of sniffing, which indicates that the animal accelerates or slows down breathing in presence of the odor.

According to another embodiment, there is a method to test discrimination of at least two different odorants by an animal, in which at least a mean instantaneous sniffing frequency F_{sniff} of a sniffing response of the animal is determined by the calculator unit 6 of the device 1 for each of the respective different odorants in the selected odorant air stream 41, wherein the respective mean instantaneous sniffing frequencies F_{sniff} associated having been determined for the respective different odorants are compared one to another to indicate non-discrimination of the animal between the different odorants when the respective mean instantaneous sniffing frequencies F_{sniff} are in a same determined range and to indicate discrimination of the animal between the different odorants when the respective mean instantaneous sniffing frequencies F_{sniff} are not in the same determined range. According to another embodiment, there is a method to test discrimination of at least two different odorants by an animal, in which the mean volume of sniffing MAUC of a sniffing response of the animal is determined by the calculator unit 6 of the device 1 for each of the respective different odorants in the selected odorant air stream 41, wherein the respective mean volume of sniffing MAUC having been determined for the respective different odorants are compared one to another to indicate non-discrimination of the animal between the different odorants when the respective mean volume of sniffing MAUC are in the same determined range and to indicate discrimination of the animal between the different odorants when the respective mean volume of sniffing MAUC are not in the same determined range. The invention enables the determination of whether two distinct odorant substances 42 can be olfactively discriminated by the animal or not, as shown in example 2 below. This enables the evaluation of whether two odorant products 42 are perceived by the animal as having similar odors. This may be useful to analyse whether modifications of a process for manufacturing a product have notable effects on the odor of a pet food.

In an embodiment shown on figure 2, the intermediate opening 23 and the air exit port 22 are situated at the bottom end 28 of receptacle 2. Consequently, the measurements of variations of air flow at the air exit port 22 by the means 3 measure the air flow caused by the nose of the animal, which is present near the intermediate opening 23 and near the bottom end to sniff the odor introduced at the intermediate opening 23.

In experiments with lickometers, odorants are provided via the liquid substance and therefore, the result is a measure of retro-nasal and taste effects. With the device 1 according to the invention, mainly the ortho-nasal effect of odorants is measured.

In an embodiment shown on figures 1 to 3, the calculator unit 6 is configured to send the selected odorant air stream 41 in the odor line 4 towards the cavity 25 of the receptacle 2 at least after a first prescribed duration T1 after the instant of detection of the presence of the animal by the presence sensor 5. The first prescribed duration T1 may be for example higher than or equal to 0.1 second and lower than or equal to 5 seconds. For example, the first prescribed duration T1 may be equal to 1.5 seconds. The first prescribed duration T1 serves to balance the air pressure and to ensure the animal is breathing normally when the odor is presented in the receptacle 2 through the odor line 4. For example, the calculator unit 6 is configured to send the selected odorant air stream 41 in the odor line 4 towards the cavity 25 of the receptacle 2, a predetermined duration DD (comprised between 0 and 1 second and for example equal to 0.25 second) after the instant when an inspiration or expiration of the animal is detected (differential air pressure value ΔP supplied by the air pressure sensor 32 higher than a first positive threshold or lower than a second negative threshold) by the air pressure sensor 32 at least after the first prescribed duration T1 after the instant of detection of the presence of the animal by the presence sensor 5.

In an embodiment shown on figures 1 to 3, the calculator unit 6 is configured to validate measurements corresponding to the detection of presence of the animal by the presence sensor 5 for at least a second prescribed duration T2, as being representative of a valid trial. The second prescribed duration T2 may be for example higher than or equal to 1 second and lower than or equal to 30 seconds. For example, the second prescribed duration T2 may be equal to 10 seconds. The measurements corresponding to the detection of presence of the animal by the presence sensor 5 during a duration lower than the second prescribed duration T2 are disregarded by the calculator unit 6 as being not representative of a valid trial.

In another embodiment, the calculator unit 6 is configured to send the selected odorant air stream 41 in the odor line 4 towards the cavity 25 of the receptacle 2, a predetermined duration DD (comprised between 0 and 1 second and for example equal to 0.25 second) after the instant when an inspiration or expiration of the animal is detected (differential air pressure value ΔP supplied by the air pressure sensor 32 higher than a first positive pressure amplitude threshold THR1 or lower than a second negative pressure amplitude threshold THR2) by the air pressure sensor 32.

In an embodiment, the calculator unit 6 may be connected to a computer, which is configured to emit a first continuous tone during the second prescribed duration T2 or as long as the presence of the animal is detected by the presence sensor 5. After the second prescribed duration T2, the computer is configured to stop emitting of the first continuous tone and to emit a second tone (for example a chirp) different from the first continuous tone. When the presence of the animal is not detected anymore by the presence sensor 5 before the second prescribed duration T2, the computer is configured to stop emitting of the first continuous tone.

In an embodiment shown on figures 1 to 3, the calculator unit 6 is configured to stop sending of the selected odorant air stream 41 in the odor line 4 towards the cavity 25 of the receptacle 2 after a third prescribed duration T3 after the instant of detection of absence of the animal. The third prescribed duration T3 may be for example higher than or equal to 0.1 second and lower than or equal to 5 seconds. For example, the third prescribed duration T3 may be equal to 1 second. The trial is then terminated.

In an embodiment shown on figures 1, 3 and 4, the presence sensor 5 is situated to detect the presence of the animal in front of the front opening 21, i.e. when the animal inserts its nose in the receptacle 2 through the front opening. The presence sensor 5 can be situated in front of the front opening 21 or behind the front opening 21 and is fixed relative to the receptacle 2. In an embodiment shown on figures 1, 3 and 4, the presence sensor 5 comprises an infrared beam sensor 50 able to detect the presence of the animal, i.e. animal nose, when the animal or animal nose breaks the infrared beam 53 emitted by the sensor 50. In an embodiment shown on figures 1, 3 and 4, the infrared beam sensor 50 comprises an emitter 51 to emit the infrared beam 53 situated in front of the front opening 21 towards a detector 52 to detect the infrared beam 53. The emitter 51 is connected through the wires 510 to the calculator unit 6 to be controlled by the latter and to send its measurement to the latter. The detector 52 connected through the wires 520 to the calculator unit 6 to be controlled by the latter and to send its measurement to the latter. The detector 52 faces the emitter 51 in front of the front opening 21. For example, the emitter 51 is situated at a first front outer side 211 in view of the front opening and the detector 52 is situated at a second front outer side 212 in view of the front opening, with the second front outer side 212 (for example being on the right in view of the front opening 21) being opposite to the first front outer side 211 (for example being on the left in view of the front opening 21) in view of the front opening 21. When the emitter 51 is in an operating state, the emitter 51 emits the infrared beam 53 directed towards the detector 52. When the detector 52 detects the infrared beam 53, the infrared beam sensor 50 is in a state of detection of absence, i.e. of the animal. When in the operating state of the emitter 51 the infrared beam 53 is not detected by the detector 52, the infrared beam sensor 50 is in a state of detection of presence, i.e. of the animal.

Of course, the above applies also to the infrared beam sensor 50' having the emitter 51' and the detector 52' situated so as to emit the infrared beam 53' in the receptacle 2 as shown on figures 1 and 3 (by replacing 50 by 50', 51 by 51', 52 by 52' and 53 by 53') in another embodiment. In this embodiment, the infrared beam sensor 50' having the emitter 51' and the detector 52' is for example attached under the front wall 101 as shown on figures 1 and 3 or to the external side of the receptacle 2. In these cases, the wall 24 of the receptacle 2 may be transparent, so that the infrared beam 53' may be sent through the transparent wall 24 by the emitter 51' to the detector 52'. In these cases, no presence sensor 5 is provided on the front wall 101.

In other embodiments which are described below, the presence sensor 5 (then called second presence sensor 5b) may use the measurement of air pressure in the air exit port 22, supplied by the air flow sensor 3, as described below. In this case, the presence sensor 5, 5b is embodied in the calculator unit 6 and is configured to detect the presence of the animal when variations of the air flow in the air exit port 22 is representative of inspiration and/or expiration of the animal. In an embodiment, the air flow sensor 3 may comprise an air flow pressure sensor 32 to measure a pressure of the air flow in the air exit port 22. The presence sensor 5, 5b is at least partly embodied in the calculator unit 6 and is configured to detect the presence of the animal when the pressure of the air flow in the air exit port 22 has a pressure amplitude which is above a predetermined pressure amplitude threshold THR1 or THR2.

In an embodiment, no infrared beam sensor 50 or 50' may be provided in the presence sensor 5, 5b.

In another embodiment, a first presence sensor 5a comprising an infrared beam sensor 50 or 50' described above and a second presence sensor 5b are provided as presence sensors 5. The first presence sensor 5, 5a comprises an infrared beam sensor 50 (or 50') to project an infrared beam 53 (or 53') situated in front of the receptacle 2 or in the receptacle 2. The infrared beam sensor 50 (or 50') comprises an emitter 51 (or 51') to emit the infrared beam 53 (or 53') and a detector 52 (or 52') to detect the infrared beam 53 (or 53') The first presence sensor 5, 5a is configured to detect a first event when the emitter 51 (or 51') is in an operating state, in which the emitter 51 (or 51') emits the infrared beam 53 (or 53') directed to the detector 52 (or 52') and the infrared beam 53 (or 53') is not detected by the detector 52 (or 52'). The air flow sensor 3 comprises an air flow pressure sensor 32 to measure a pressure of the air flow in the air exit port 22. The second presence sensor 5, 5b is at least partly embodied in the calculator unit 6 and is configured to detect the presence of the animal when the pressure of the air flow in the air exit port 22 has a pressure amplitude which is above a predetermined pressure amplitude threshold THR1 or THR2 after a predetermined duration DD after the first event.

In an embodiment shown on figure 1, the air flow sensor 3 comprises a differential air pressure sensor 32 connected to the air exit port 22. The air flow sensor 3 is of pneumo-tachometer type and measures by the differential air pressure sensor 32 a differential air pressure value ΔP indicating inspiration and expiration through the air exit port 22. The differential air pressure sensor 32 is for example connected by a first air line 33 and by a second air line 34 to an air flow section 35, which is connected to the air exit port 22 downstream of the latter. The differential air pressure sensor 32 supplies a measurement being a differential air pressure value ΔP (which is for example instantaneous) of air flowing through the air exit port 22, which is a difference between air flow pressure P₃ measured by it in the first air line 33 and air flow pressure P₄ measured by it in the second air line 34, i.e. ΔP = P₃ - P₄. The first air line 33 is upstream from the second air line 34 and downstream from the air exit port 22 (wherein the downstream direction is direction of air flow going from the front opening 21 to the air exit port 22). The first air line 33 is at a distance from the second air line 34. The air lines 33 and 34 are separate from the odor line 4. A mesh 31 (or membrane having holes allowing air flow through it) is situated cross the air flow section 35 situated between the first air line 33 and the second air line 34, in order to create a difference of pressure between the first air line 33 and the second air line 34, in order to know from the air pressures P₃ , P₄ measured respectively in air lines 33 and 34 by the differential air pressure sensor 32, whether the air flow in the air exit port 22 is an inspiration (pressure of air flow in the first air line 33 higher than pressure of air flow in the second air line 34) coming from the animal or an expiration (pressure of air flow in the first air line 33 lower than pressure of air flow in the second air line 34) coming from the animal. In an embodiment, the differential air pressure sensor 32 is connected through first wires 320 or other means to the calculator unit 6 to send its measurement of the differential air pressure value (for example instantaneous) ΔP of air flowing through the air exit port 22 to the calculator unit 6. In an embodiment, the differential air pressure sensor 32 may be part of the calculator unit 6. All air lines may be a flexible tubing.

In an embodiment, the calculator unit 6 (what concerns the computer unit 6 may be done also by the computer connected to the calculator unit 6 in the disclosure) comprises processing means or a processor or a calculator to calculate the parameters indicated below.

In an embodiment, the calculator unit 6 calculates or determines or identifies from the differential air pressure value ΔP of air flowing through the air exit port 22, having measured by the differential air pressure sensor 32, as sniffing response at least one of :
- zero values ΔP₀ of the differential air pressure value ΔP,
- zero crossings ΔP_{0crossing} of the differential air pressure value ΔP (the zero crossings are assumed to indicate a change from inspiration to expiration or vice versa),
- peaks PE (positive or negative) of the differential air pressure value ΔP,
- the heights HPE of the peaks as being the respective absolute values of the peaks of the differential air pressure value ΔP (the height of peaks is also called pressure amplitude above),
- the number NPE of peaks of the differential air pressure value ΔP,
- the time Tr to reach each peak PE as the time elapsing from the preceding zero crossing ΔP_{0crossing} to this peak PE.

In an embodiment, the calculator unit 6 calculates as a sniffing response a mean instantaneous sniffing frequency F_{sniff} (also called sniffing frequency F_{sniff} or the sniff frequency F_{sniff} below) in the receptacle 2 from the differential air pressure values ΔP of air flowing through the air exit port 22, having been measured by the differential air pressure sensor 32. The mean instantaneous sniffing frequency F_{sniff} is a mean of frequency of peaks of the differential air pressure value ΔP.

Embodiments below are described to calculate the following trial parameters as a sniffing response, which are used in the examples described below.

In an embodiment, in case of a positive peak PE of the differential air pressure value ΔP situated between two consecutive zero values of ΔP, the calculator unit 6 identifies this peak as being an inspiration peak PEᵢₙₛₚ (corresponding to a single episode of inspiration). In an embodiment, in case of a negative peak PE of the differential air pressure value ΔP situated between two consecutive zero values of ΔP, the calculator unit 6 identifies this peak as being an expiration peak PEₑₓₚ (corresponding to a single episode of expiration). If the peak flow rate (also measured by the air pressure sensor 32) is less than a first positive predetermined value PE1 (for example 10 L/min) or greater than a second negative predetermined value PE2 (for example -10 L/min for expiration), this flow rate and the corresponding peak of ΔP is rejected by the calculator unit 6 (for example as data in which there was an insufficient seal between the muzzle of the animal and mask 2). The rejections may be made a posteriori, i.e. after having stored all the peaks PE by the calculator unit 6 in a memory.

In an embodiment, the calculator unit 6 calculates an instantaneous inspiration frequency Fᵢₙₛₜ₋ᵢₙₛₚ of each inspiration peak PEᵢₙₛₚ by identifying the time Tᵢₙₛₚ elapsed between successive inspiration peaks and calculating the reciprocal of this time Tᵢₙₛₚ as being the instantaneous inspiration frequency Fᵢₙₛₜ₋ᵢₙₛₚ, i.e. Fᵢₙₛₜ₋ᵢₙₛₚ = 1 / T_{insp.} In an embodiment, the calculator unit 6 calculates an instantaneous expiration frequency Fᵢₙₜ₋ₑₓₚ of each expiration peak PEₑₓₚ by identifying the time Tₑₓₚ elapsed between successive expiration peaks and calculating the reciprocal of this time Tₑₓₚ as being the instantaneous expiration frequency Fᵢₙₜ₋ₑₓₚ, i.e. Fᵢₙₜ₋ₑₓₚ = 1 / Tₑₓₚ. In an embodiment, instantaneous inspiration frequencies Fᵢₙₛₜ₋ᵢₙₛₚ and/or instantaneous expiration frequencies Fᵢₙₜ₋ₑₓₚ higher than a third predetermined frequency value (for example 25 Hz) were rejected by the calculator unit 6 (for example as being noise). The rejections may be made a posteriori, i.e. after having stored all the frequencies by the calculator unit 6 in a memory.

In an embodiment, the calculator unit 6 calculates the sniffing frequency F_{sniff} as being the overall mean of the instantaneous inspiration frequencies Fᵢₙₛₜ₋ᵢₙₛₚ and instantaneous expiration frequencies Fᵢₙₜ₋ₑₓₚ.

In an embodiment, the calculator unit 6 calculates an AUC (area under the curve: called also volume of sniffing) of the differential air pressure value ΔP as the area under a curve between two successive zero crossings for each peak and/or a total area TAUC under the curve of the differential air pressure value ΔP (being the sum of the absolute values of the areas AUC). In an embodiment, the calculator unit 6 determines the sniffing peak maximum Pₘₐₓ as the maximum of the heights of the peaks. In an embodiment, the calculator unit 6 determines time Tᵣ to reach each peak as the time elapsing from the preceding zero crossing to this peak. In an embodiment, the calculator unit 6 calculates the sniffing frequency F_{sniff}, the total area TAUC, the sniffing peak maximum Pₘₐₓ and the times Tᵣ to reach the peaks. In an embodiment, the calculator unit 6 calculates the minimum, the maximum, and the mean of each of: the instantaneous expiration frequencies Fᵢₙₜ₋ₑₓₚ of the expiration peaks, the instantaneous inspiration frequencies Fᵢₙₜ₋ᵢₙₛₚ of the expiration peaks, the areas AUC under the curve of the differential air pressure value ΔP, the height HPE of the peaks, the times Tᵣ to reach the peaks.

In an embodiment, the calculator unit 6 calculates a baseline sniffing frequency F_{b}. In an embodiment, the calculator unit 6 calculates a baseline sniffing frequency F_{b} by averaging the sniffing frequency F_{sniff} for each dog across all blank trials for each odorant. In an embodiment, the calculator unit 6 calculates a measure MPB_{sniff} of proportion of baseline sniffing frequency by dividing the sniffing frequency F_{sniff} for each odorant trial by the baseline sniffing frequency F_{b}. In an embodiment, the calculator unit 6 calculates a log proportion LOGMPB_{sniff} of baseline sniffing frequency by log transforming this measure MPB_{sniff} of proportion of baseline sniffing frequency. A value of zero of the log proportion LOGMPB_{sniff} of baseline sniffing frequency indicates that sniffing during the odorant trial was identical to sniffing during blank trials. In an embodiment, to evaluate whether significantly more sniffing was observed at a specific dilution, the log proportion LOGMPB_{sniff} of baseline sniffing frequency at each dilution is tested for being significantly greater than zero on a one-sided one-sample t-test. These calculations may be carried out a posteriori by a person using the calculator unit 6.

In an embodiment shown on figures 1 and 2, the device comprises, in addition to the air flow sensor 3 at least one other sensor 7 placed at the air exit port 22. The other sensor 7 is connected through third wires 70 to the calculator unit 6 to send its measurement to the latter. According to an embodiment of the invention, the device may comprise at least one other sensor placed at the air exit port 22, chosen among a thermocouple 7, a temperature sensor 7, a microphone 7, a pCO2 sensor 7 (or a capnometer 7), or others. The device may comprise a thoracic belt intended to be attached around the thorax of the animal and having a sensor 7 to record inspiration and/or expiration movement happening during the olfactory exploration, wherein the sensor 7 of the thoracic belt is connected through wires (or wirelessly through an antenna) to the calculator unit 6 to send its measurements to the latter.

For example, the air flow sensor 33 and/or differential pressure sensor 32 and/or temperature sensor 7 is (are) connected to an analog-to-digital converter (for example of 16 bits) of the calculator unit 6, which is connected to a microcontroller of the calculator unit 6, which may be connected to a computer. Of course, further sensors or measurements could be provided.

In an embodiment shown on figures 1, 5 and 6, the casing contains one or more containers 8 (as shown by the dotted lines of figure 1) containing an odorant substance 42 and an air sending unit 9 to send an air stream through the container 8 to introduce the selected odorant air stream 41 corresponding to the selected container 8 into the odor line 4, and then through the intermediate opening 23 into the inner cavity 25 of the receptacle 2. Each container 8 may be for example a jar or a flexible bag or other. Each container 8 may have two access openings 89a, 89b to connect air lines 91, 93 therein. A respective connector valve 89c, 89d may be provided on each access opening 89a, 89b and have an opened position to let air pass through the access opening 89a, 89b and a closed position not to let air flow through the access opening 89a, 89b, especially for example for a flexible bag as container 8. When using the device, even in the odor sending position and in the blank or cleaning position described below, the respective connector valve 89c, 89d is maintained in the opened position. The container 8, especially the flexible bag, may have a septum 89e closing it but enabling to introduce a needle of a syringe or others devices, in order to introduce an odorant substance 42 (gaseous or liquid) into the container 8.

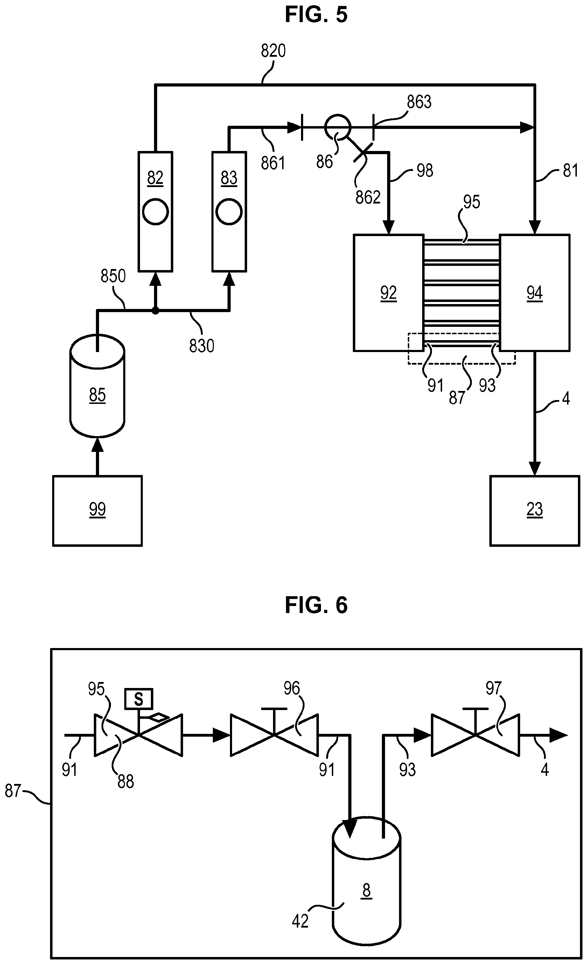

In an embodiment shown on figures 1, 5 and 6, each container 8 is connected through a first air line 91 to a first distributing module 92 (which may be a first manifold) of the air sending unit 9 and through a second air line 93 to a second distributing module 94 (which may be a manifold) of the air sending unit 9. The part 87 in dotted line of figure 5 is shown in a more detailed manner in figure 6 and forms an air connection module 87 for each container 8, comprising the elements 95, 88, 96, 91, 8, 93 and 97 described below. The first distributing module 92 comprises for each container 8 a first valve 95 (for example a solenoid valve) on the first air line 91. On the first air line 91 may be provided a check valve 96, which is normally opened in a presence of a flow of air flowing in the first air line 91 from the first distributing module 92 to the container 8 and which serves only to prevent reverse flow of air. The second distributing module 94 comprises for each container 8 a second check valve 97 on the second air line 93, wherein the second check valve 97 is normally opened in a presence of a flow of air flowing in the second air line 93 from the container 8 to the odor line 4 and serves only to prevent reverse flow of air. The odor line 4 is connected to the second distributing module 94 by the second valve 97. The first distributing module 92 is connected to an air pump 99 by at least a third air line 98, in order to send air by the air pump 99 to the third air line 98 and then to the container 8 and the odor line 4, when the valves 95, 96 and 97 are set in an opened position (odor sending position).

In an embodiment, the second distributing module 94 may be connected to the air pump 99 by at least a fourth air line 81, in order to send air by the pump 99 to the fourth air line 81 and then to the second distributing module 94 and then to the odor line 4, in order to vent the odor line 4 by bypassing the container 8 (valves 95, 96 and 97 set in a closed position not letting air passing through the first air line 91 and the second air line 93), to enable to clean the odor line 4 from odors (blank or cleaning position mentioned below).

In an embodiment, at least one rotameter 83 (as air flow rate regulator) is provided in an air line 830 connected between the air pump 99 and the first distributing module 92, to enable to set different flow rates in the air lines 830, 98, 91, 93 and then into the odor line 4 in the odor sending position. The air line 98 may be connected to the air connection module 87 and to the air line 91 through the first distributing module 92, as described below. Another rotameter 82 (as air flow rate regulator) may be provided in an air line 820 connected between the air pump 99 and the second distributing module 94, to enable to set different flow rate in the air line 81 and then into the odor line 4 in the cleaning position. The rotameter 83 and/or 82 may be connected to the air pump 99 through a filter 85 situated in an air line 850 situated upstream to the air line 830 and/or 820. The filter 85 can be a carbon and particulate filter or a cleaned charcoal filter, in order to retain dirt or other undesirable particulates from air sent to the air lines.

In an embodiment, a three-way valve 86 is provided, having a first access 861 connected to the rotameter 83, a second access 862 connected to the air line 98 and a third access 863 connected to the fourth air line 81. The three-way valve 86 may be set in a first position (odor sending position) for sending the selected odorant air stream 41 on the odor line 4, in which the first access 861 is connected to the second access 862, and a second position (blank position), in which the first access 861 is connected to the third access 863 for sending a flow of air into the fourth air line 81.

The air sending unit 9 is connected by control wires 90 to the calculator unit 6, which controls the air sending unit 9 and the selectors 88, which are formed for example by the first valves 95.

For example, in the embodiment shown, at least two containers 8 containing different odorant substances 42 producing different odors are provided and are each connected by different air lines 91 and 93 to the air sending unit 9. Any one of the containers 8 can be selected on the air lines 91 and/or on the air lines 93 by selectors 88 (formed for example by the valves 95) provided in the air sending unit 9 to receive the air stream from the air sending unit 9, so that the air stream mixed with the selected odor produced by the selected container 8 is sent to the odor line 4.

In an embodiment, the calculator unit 6 is configured to control the valves 95 and the three-way valve 86. In an embodiment, the rotameter(s) 83 and/or 82 may be controlled manually by actuating mechanic control members of it in order to vary the air flow rate in the air lines. In another embodiment, the valves 95, 97 and the three-way valve 86 may be controlled manually by actuating mechanic control members of them.

The odor may be a food odor. The odorant substance 42 may be a piece of food, or a solution of an odorant substance (such as an aqueous solution or oil or a solid substance or a powder) or at least one volatile organic compound or a food odorant substance or any physical support (such as any object or material...), or a pet food, or mixtures of them. The concentration of the odorant substance 42 in the container 8 may have been determined beforehand. The volume of air contained in each container 8 may be determined beforehand. Different odors or different odorant substances 42 or different concentrations of a same odorant substance 42 may be provided in the container(s) and may be tested. For example, the air flow rate of the air stream sent by the air sending unit 9 in the air line 81 can be 1 liter per minute, the air flow rate of the air stream sent by the air sending unit 9 in each air line 91 and 93 may be 0.5 liter per minute. Of course, other values of the air flow rates are possible.

The calculator unit 6 may be connected to a computer to control the trials operated by the device 1. For example, the computer may be configured to control the air sending unit 9 and the selectors 88 to control whether or not an odor is presented by the odor line 4 in the receptacle 2 and/or which one of several odors is presented by the odor line 4 in the receptacle 2 and/or at which concentration or dilution the odor is presented by the odor line 4 in the receptacle 2. The calculator unit 6 may have means to analyze the measurements supplied by the measurements means 3 and by the presence sensors 5 to the calculator unit 6. The calculator unit 6 and/or the computer may comprise a memory to store the instructions sent to the air sending means 9 and/or the selection means 93 and/or the detections of presence by the presence sensor 5 and/or the detections of absence by the presence sensor 5, and/or an identification of the odorant substance 42 which odor is sent in the selected odorant air stream 41 and the measurements 3 supplied by the air flow sensor.

In an embodiment, prior to the start of testing, the air flow sensor 3 was calibrated by passing a known flow rate through the receptacle measured by a rotameter 82 or 83. In an embodiment, the differential pressure readings (ΔP) from the differential pressure sensor 32 were transformed to flow rates in the calculator unit 6 (for example in a proportional manner). Temperature readings from the temperature sensor 7 were transformed to temperature readings following the manufacturer's recommendations.

### Example 1- Engage attention threshold

### Dog panel

A cohort of eight dogs of diverse breeds, aged 1-6 years, housed at a facility were involved in the study. Dogs were pair-housed in pens and received twice daily play time, walks, and social enrichment.

### Dog training phase

Dogs were encouraged to place their nose within the mask. A tone is emitted during the duration the dog keeps its nose in the mask. Once the dog reached a previously determined duration, another sound (such a "chirp") indicated that he will be rewarded (for example with treats). Across successful trials, the duration is incremented. If the dog exits the mask prior to the predetermined duration DD, the tone would simply stop, and no treat was given. The required duration was then decreased to the previous validated duration. Once « nose in » time exceeds 10 seconds, dogs are qualified to start the tests.

Prior to the start of the study, dogs were given a refresher training session to insure dogs' ability to maintain their nose in the mask for 10 s.

### Dog testing phase

In this testing phase, two odorants products were tested: odorant OD1 (pure molecule) and odorant OD2 (food product) as odorant substance 42 (called below odorant) in different volume dilutions DIL by using the device according to the invention.

Once daily, dogs were given one brief testing session of 10 trials. In a single daily session, 5 dilution steps were presented with intervening blank trials (no odorant) providing 10 total trials. The most diluted odorant was presented first and concentration was increased 10-fold across odor trials. Thus, dogs were presented dilutions ranging from 10⁻¹² to 10⁻³ Mol/L for OD1 over two consecutive days. For OD2, dogs were presented dilutions ranging from 10⁻¹² to 10⁻¹ volume/volume dilution and undiluted condition over three consecutive days (only 3 dilutions the last day)

### Data treatment & Results

Pressure traces of sensor 32 were calibrated such that a value of zero indicated no air flow. The calculation of the trial parameters described above was made by the device according to the invention for odorant OD1 and odorant OD2 in the trials.

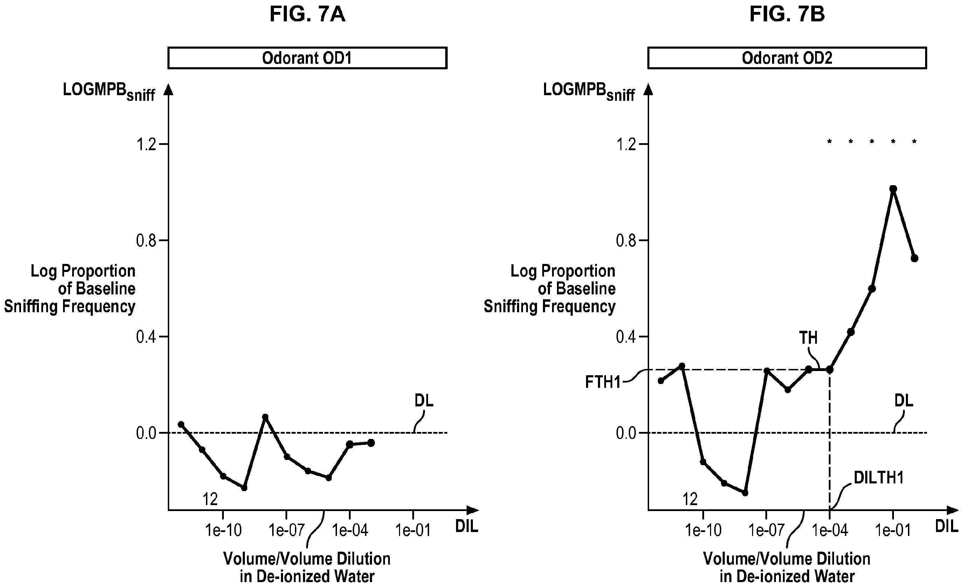

Figures 7A and 7B show the log proportion LOGMPB_{sniff} of baseline sniffing frequency in ordinates as a function of volume dilution DIL of the odorant substance 42 (being an odorant OD1 for figure 7A or being an odorant OD2 for figure 7B) in deonized water in abscissas. Each point shows the mean. The dashed line DL indicates sniffing during blank trials (value of zero of the log proportion LOGMPB_{sniff} of baseline sniffing frequency). In the figure 7A and 7B, * indicates significantly more sniffing (p-value ≤ 0.05 on a one-sample t-test) compared to the respective blank trials.

Figure 7A indicated that the sniffing response LOGMPB_{sniff} remained low for odorant OD1 up to the last dilution level. Odorant OD1 was selected from a literature review, known to be perceived by dogs. Figure 7A shows that dogs do not engage attention toward this specific odorant OD1.

Figure 7B shows that dogs showed increasing sniffing (higher sniffing response LOGMPB_{sniff} indicated by *) for the odorant OD2 as concentration of the odorant substance 42 increased (volume dilution DIL decreased). Indeed, Figure 7B shows that the sniffing response LOGMPB_{sniff} for the odorant OD2 starts to increase above the first determined threshold FTH1 (here in this example equal to approximately 0.3) of LOGMPB_{sniff}, when the volume dilution DIL is equal or higher than a certain second threshold DILTH1 of the volume dilution DIL, which can be a minimal concentration threshold DILTH1 (here in this example equal to approximately 10⁻⁴) of the volume dilution DIL. This second threshold DILTH1 (here in this example equal to approximately 10⁻⁴) of the volume dilution DIL corresponds to an engage attention threshold of the animals. Even if the sniffing response seemed to decrease for the last and final trial (undiluted) compared to the previous trials the difference is not significant (t = 1.1481, df = 17.389, p-value = 0.2665). In the example 1, p-value is a probability value for a given statistic model, in which the hypothesis to obtain the same value of the log proportion LOGMPB_{sniff} or a more extreme value of the log proportion LOGMPB_{sniff} assuming there is no difference. df is a degree of freedom. The degree of freedom df refers to the number of parameters which can vary in an independent manner. t is the t-value of the Student test. t corresponds to the size of the effect of the relative difference concerning the variation of sample distribution (i.e. a number of times of the standard error).

This example 1 shows that through device 1 according to the invention it is possible to determine engage attention thresholds of a given odorant.

### Example 2 - Habituation-Dishabituation Discrimination

In this example 2, the dog panel and the dog training phase are the same as in example 1 above.

### Dog testing phase

The dogs' spontaneous discrimination was compared between two commercial dog foods (X and Y) and a mix of both as odorant substance 42, by using the device of the invention.

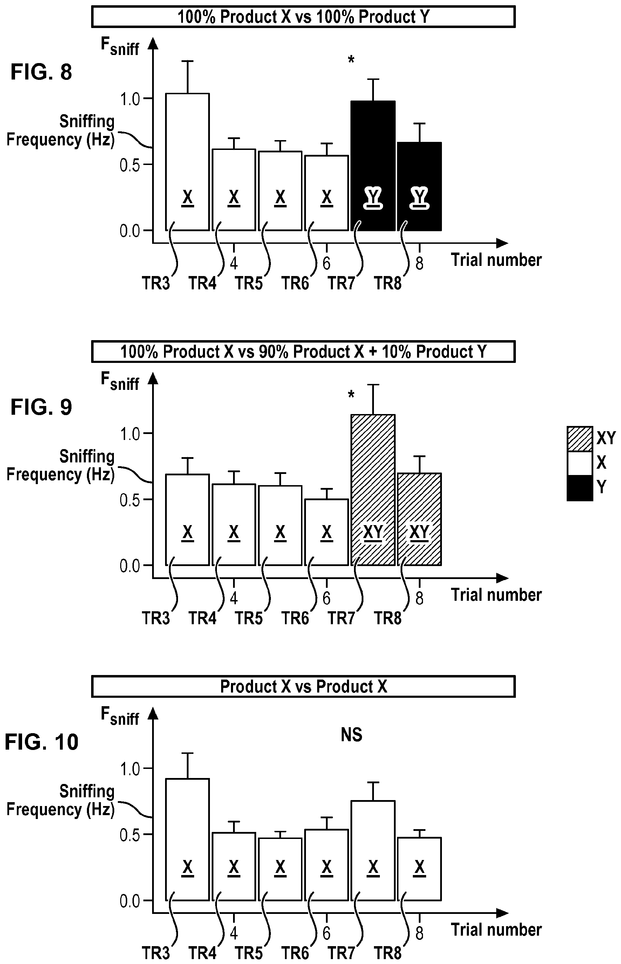

In the trials, the comparisons were as follows for the odorant substance 42 (called below odorant):
- 100% by weight of Product X (indicated as X in trials TR3, TR4, TR5, TR6 in figure 8) vs 100% by weight of Product Y (indicated as Y in trials TR7 and TR8 in figure 8),
- 100% by weight of Product X (indicated as X in trials TR3, TR4, TR5, TR6 in figure 9) vs a mix of 90% by weight of Product X+10% by weight of Product Y (indicated as XY in trials TR7 and TR8 in figure 9),
- 100% by weight of Product X (indicated as X in trials TR3, TR4, TR5, TR6 in figure 10) vs 100% by weight of Product X (indicated as X in trials TR7, TR8 in figure 10), serving as control condition.

The first product X in each comparison served as the habituating odor stimulus and the second product served as the dis-habituating odor stimulus.

The mix XY was tested to evaluate dogs' ability to spontaneously discriminate between a food and the same food slightly adulterated with the odor of a different product.

Control condition was conducted to insure dishabituation was due to the odorant change and not an unintentional change in the experiment. In this control condition the same odorant (Product X), served as the habituating and dishabituating odorants although they were treated as separate odorants and placed in separate jars 8.

Dogs were given one odor comparison per day which was comprised of several trials (see Table 1 below). Table 1 shows the trial layout and the trial order for each daily session. Dogs were first presented with two blank trials, followed by the habituating stimulus for four trials, and the dishabituating stimulus for two trials. The order of the odor comparisons tested was randomized across dogs. All dogs completed each comparison once, which was then replicated.

**Table 1.**

| Trial TR1 | Trial TR2 | Trial TR3 | Trial TR4 | Trial TR5 | Trial TR6 | Trial TR7 | Trial TR8 |
|---|---|---|---|---|---|---|---|
| Blank | Blank | Habit odor | Habit odor | Habit odor | Habit odor | Dishabit odor | Dishabit odor |

### Data treatment & Results

Pressure traces of sensor 32 were calibrated such that a value of zero indicated no air flow. The calculation of the trial parameters leading to the sniffing frequency F_{sniff} described above was made by the device 1 according to the invention for the above-mentioned odorant substances 42 in the trials. Blank trials served as 'warm up' trials to get the dog working on the task before presenting them with the odor and thus the data coming from these trials were not used.

The sniffing frequency F_{sniff} was calculated for each trial. To identify whether significant dishabituation occurred when the odorant was changed, analyses were focused on the differences in sniffing frequency F_{sniff} between trials TR6 and TR7 (change of habituation stimulus to dishabituation stimulus). The sniffing frequencies F_{sniff} for each trial were log transformed to calculate a log value LOGF_{sniff} of the sniffing frequencies F_{sniff} to normalize the data. A linear mixed-effect model which included random intercepts for participants was fit. Fixed effects included the odor comparison, the trial type (habituation or dishabituation) and their interaction. LSMeans with a post-hoc correction were used to compare the log values LOGF_{sniff} of the sniffing frequencies F_{sniff} between the habituation and dishabituation trial for each comparison.

Figures 8, 9 and 10 show sniffing frequency F_{sniff} (+/- standard error SE) (expressed in Hz) for the last six trials, i.e. trial TR3, trial TR4, trial TR5, trial TR6, trial TR7, trial TR8 having the above-mentioned odorants. * indicates a significantly higher sniffing frequency F_{sniff} (p<0.05 and LSMeans t-test) for trial TR7 in figures 8 and 9, i.e. the sniffing frequency F_{sniff} of trial TR7 is above a determined range RG (for example RG is equal to +/- 30 %) of the sniffing frequency F_{sniff} of the preceding trial TR6; this value of RG has been determined with 95% of the confidence interval of the sniffing frequency F_{sniff} of the preceding trial TR6, if the value of RG is not inside the confidence interval (or inside the 2 standard deviations around the mean value shown on the figure), the products can be considered as statistically different). This indicates discrimination between trial TR7 and trial TR6 of figures 8 and 9. Figures 8, 9 show that dogs showed significant dishabituation between products X and Y (t=2.56, df=110, p=0.01) and product X and the mixture XY (t=3.78, df=110, p<0.01). This indicates discrimination.

No difference was observed on the control trial (product X vs product X: t=1.41, df=110, p=0.16) of figure 10. NS indicates a not significant result (p>0.05) in figure 10, i.e. the sniffing frequency F_{sniff} of trial TR7 is within a determined range RG (for example +/- 30 %) of the sniffing frequency F_{sniff} of the preceding trial TR6. This shows no discrimination between trial TR7 and trial TR6 of figure 10. In this example 2, p-value is a probability value for a given statistic model, in which the hypothesis to obtain the same value of the sniffing frequency F_{sniff} or a more extreme value of the sniffing frequency F_{sniff} than the one observed assuming there is no difference between trials, df is a degree of freedom. The degree of freedom df refers to the number of parameters which can vary in an independent manner. t is the t-value of the Student test. t corresponds to the size of the effect of the relative difference concerning the variation of sample distribution (i.e. a number of times of the standard error).

The device 1 according to the invention allows a rapid probing of large numbers of animals to evaluate whether dogs spontaneously discriminate between food odors. This is useful when questions arise as to whether changes in production procedures have a noticeable effect on the aroma of the food. Although this could be evaluated using dogs trained in an operant discrimination test, dogs explicitly rewarded and trained to discriminate aromas may show discrimination between very subtle differences that may not represent untrained (or spontaneous) discrimination. This procedure allows rapid probing of untrained dogs' odor perception.

Of course, the aspects, embodiments, features, possibilities and examples of the invention mentioned above may be combined one with another or may be selected independently one from another.

## Claims

1. A device (1) for testing sniffing of an animal, the device (1) comprising :
a receptacle (2) having a front opening (21) for receiving air flow produced by the animal,
**characterized in that** the device (1) further comprises an air flow sensor (3), which is configured to measure air flow in an air exit port (22) of the receptacle (2),
an odor line (4), able to introduce a selected odorant air stream (41) into the receptacle (2),
a calculator unit (6) for determination of a sniffing response of the animal for the selected odorant air stream (41) based on the air flow measured by the air flow sensor (3),
at least one presence sensor (5), which is configured to detect the presence of the animal,
the calculator unit (6) is configured to command sending of the selected odorant air stream (41) in the odor line (4) towards the receptacle (2) as a response to the fact that the presence sensor (5) has detected the presence of the animal.

2. The device according to claim 1, wherein the presence sensor (5, 5a) comprises an infrared beam sensor (50) to project an infrared beam (53) situated in front of the receptacle (2) or in the receptacle (2),
wherein the infrared beam sensor (50) comprises an emitter (51) to emit the infrared beam (53) and a detector (52) to detect the infrared beam (53),
wherein the presence sensor (5, 5a) is configured to detect the presence of the animal when the emitter (51) is in an operating state, in which the emitter (51) emits the infrared beam (53) directed to the detector (52) and the infrared beam (53) is not detected by the detector (52).

3. The device according to claim 1, wherein the presence sensor (5, 5b) is embodied in the calculator unit (6) and is configured to detect the presence of the animal when variations of the air flow in the air exit port (22) correspond to inspiration and/or expiration of the animal.

4. The device according to claim 1, wherein the air flow sensor (3) comprises an air flow pressure sensor (32) to measure a pressure of the air flow in the air exit port (22),
wherein the presence sensor (5, 5b) is at least partly embodied in the calculator unit (6) and is configured to detect the presence of the animal when the pressure of the air flow in the air exit port (22) has a pressure amplitude which is above a predetermined pressure amplitude threshold (THR1, THR2).

5. The device according to claim 1, wherein a first presence sensor (5a) and a second presence sensor (5b) are provided as presence sensors (5),
wherein the first presence sensor (5, 5a) comprises an infrared beam sensor (50) to project an infrared beam (53) situated in front of the receptacle (2) or in the receptacle (2), wherein the infrared beam sensor (50) comprises an emitter (51) to emit the infrared beam (53) and a detector (52) to detect the infrared beam (53), wherein the first presence sensor (5, 5a) is configured to detect a first event when the emitter (51) is in an operating state, in which the emitter (51) emits the infrared beam (53) directed to the detector (52) and the infrared beam (53) is not detected by the detector (52),
wherein the air flow sensor (3) comprises an air flow pressure sensor (32) to measure a pressure of the air flow in the air exit port (22),
wherein the second presence sensor (5, 5b) is at least partly embodied in the calculator unit (6) and is configured to detect the presence of the animal when the pressure of the air flow in the air exit port (22) has a pressure amplitude which is above a predetermined pressure amplitude threshold (THR1, THR2) after a predetermined duration (D) after the first event.

6. The device according to any one of the preceding claims, wherein the odor line (4) is connected to an intermediate opening (23) of the receptacle (2), situated between the front opening (21) and the air exit port (22).

7. The device according to any one of the preceding claims, wherein the air flow sensor (3) is of pneumo-tachometer type and measures a differential air pressure value (ΔP) indicating inspiration and expiration through the air exit port (22),
the calculator unit (6) or a computer connected to the calculator unit (6) is configured to determine as sniffing response a mean instantaneous sniffing frequency (F_{sniff}) being a frequency of peaks of the differential air pressure values (ΔP) measured by the air pressure sensor (3) through the air exit port (22).

8. The device according to claim 7, wherein the calculator unit (6) or a computer connected to the calculator unit (6) is configured to determine as sniffing response:
- inspiration peaks and expiration peaks as peaks of the differential air pressure values (ΔP) in the air exit port (22),
- an instantaneous inspiration frequency (Fᵢₙₛₜ₋ᵢₙₛₚ) of each inspiration peak by identifying the time Tᵢₙₛₚ elapsed between successive inspiration peaks and calculating the reciprocal of this time Tᵢₙₛₚ as being the instantaneous inspiration frequency (Fᵢₙₛₜ₋ᵢₙₛₚ),
- an instantaneous expiration frequency (Fᵢₙₜ₋ₑₓₚ) of each expiration peak by identifying the time Tₑₓₚ elapsed between successive expiration peaks and calculating the reciprocal of this time Tₑₓₚ as being the instantaneous expiration frequency (Fᵢₙₜ₋ₑₓₚ),
- the mean instantaneous sniffing frequency (F_{sniff}) or frequency of peaks as being the overall mean of the instantaneous inspiration frequencies (Fᵢₙₛₜ₋ᵢₙₛₚ) and instantaneous expiration frequencies (Fᵢₙₜ₋ₑₓₚ).

9. The device according to any one of the preceding claims, wherein the air flow sensor (3) is of pneumo-tachometer type and measures a differential air pressure value (ΔP) indicating inspiration and expiration through the air exit port (22),
the calculator unit (6) or a computer connected to the calculator unit (6) is configured to determine as sniffing response :
zero crossings of the differential air pressure value (ΔP) measured by the air pressure sensor (3),
peaks of the differential air pressure value (ΔP) measured by the air pressure sensor (3),
a volume of sniffing being an area (AUC) between two successive zero crossings for each peak of the differential air pressure values (ΔP) measured by the air pressure sensor (3),
a mean volume of sniffing (MAUC) being a mean of the volume of sniffing of the peaks.

10. The device according to any one of the preceding claims, wherein the device (1) further comprises at least a container (8) containing an odorant substance (42) and an air sending unit (9) to send an air stream through the container (8) and to introduce the selected odorant air flow (41) coming from the container (8) into the odor line (4).

11. The device according to claim 10, wherein the device (1) comprises at least two respective containers (8) containing odorant substances (42), wherein the air sending unit (9) comprises selectors (88) enabling selection of which of the containers (8), called selected container (8), the air sending unit (9) sends air to introduce the selected odorant air flow (41) coming from the selected container (8) into the odor line (4).

12. The device according to claim 10 or 11, wherein the device (1) comprises an air flow rate regulator (82, 83) to enable to set different air flow rates in the odor line (4).

13. The device according to any one of the preceding claims, wherein the calculator unit (6) is configured to send the selected odorant air stream (41) in the odor line (4) to the receptacle (2) after at least a first prescribed duration (T1) after the instant of detection of the presence of the animal by the presence sensor (5).

14. A method to determine a minimal concentration threshold of an odorant, at which an animal shows interest, in which at least a mean instantaneous sniffing frequency (F_{sniff}) of a sniffing response of the animal and/or a mean volume of sniffing (MAUC) of a sniffing response of the animal is determined by the calculator unit (6) of the device (1) according to any one of the preceding claims for different concentrations of the odorant in the selected odorant air stream (41), wherein the minimal concentration threshold (DILTH1) of the odorant is determined by a concentration of odorant above which the mean instantaneous sniffing frequency (F_{sniff}) and/or the mean volume of sniffing (MAUC) increases above a first determined value (FTH1) or decreases below a second determined value (FTH2).

15. A method to test discrimination of at least two different odorants by an animal, in which at least a mean instantaneous sniffing frequency (F_{sniff}) of a sniffing response of the animal and/or a mean volume of sniffing (MAUC) of a sniffing response of the animal is determined by the calculator unit (6) of the device (1) according to any one of the preceding claims for each of the respective different odorants in the selected odorant air stream (41),
wherein the respective mean instantaneous sniffing frequencies (F_{sniff}) having been determined for the respective different odorants are compared one to another to indicate non-discrimination of the animal between the different odorants when the respective mean instantaneous sniffing frequencies (F_{sniff}) are in a same determined range (RG) and to indicate discrimination of the animal between the different odorants when the respective mean instantaneous sniffing frequencies (F_{sniff}) are not in the same determined range (RG) and/or
the respective mean volume of sniffing (MAUC) having been determined for the respective different odorants are compared one to another to indicate non-discrimination of the animal between the different odorants when the respective mean volume of sniffing (MAUC) are in a same determined range (RG) and to indicate discrimination of the animal between the different odorants when the respective mean volume of sniffing (MAUC) are not in the same determined range (RG).
